# EUROPEAN PATENT APPLICATION

(11) **EP 4 825 401 A2**
(43) Date of publication of application: **21.10.2026**
(21) Application number: 26203384.8
(22) Date of filing: 22.06.2022
(51) Int. Cl.: A61L 27/52

(54) **CROSS-LINKING POLYSACCHARIDE WITH FIBROIN AND USES OF THE OBTAINED MATERIAL**

(30) Priority: 23.06.2021 EP 21181097
(62) Document number(s) of the earlier application(s) in accordance with Art. 76 EPC: 22735878.5 / 4 359 021
(71) Applicant: Merz Pharma GmbH & Co. KGaA, 60318 Frankfurt am Main (DE)
(72) Inventor: Vukicevic, Radovan, 60486 Frankfurt am Main (DE); Drabe, Colin, 60322 Frankfurt (DE); Plitt, Patrick, 61352 Bad Homburg (DE); Neubauer, Jens, 63695 Glauburg (DE)
(74) Representative: Jacobi, Markus Alexander

(57) **Abstract**

The invention proposes a method for preparing a cross-linked material, comprising forming amide bonds that conjugate one or more fibroin moieties with one or more polysaccharide moieties. The invention further relates to a cross-linked material obtainable from such method and to an injectable composition comprising the obtained cross-linked material. The cross-linked material can be a hydrogel and/or a super-volumizer and can be used in cosmetic and pharmaceutical applications.

## Description

The invention relates to a method for preparing a cross-linked material, comprising forming amide bonds that conjugate one or more fibroin moieties with one or more polysaccharide moieties. The invention further relates to a cross-linked material obtainable from such method and to an injectable composition comprising the obtained cross-linked material. The cross-linked material can be a hydrogel and/or a super-volumizer and can be used in cosmetic and pharmaceutical applications.

Facial and body re-shaping is of increasing interest. For example, filling of wrinkles of face and/or body, rejuvenation of the skin, breast reconstruction or augmentation, or soft-tissue augmentation of other kind is regularly of interest. In order to avoid the need of surgical interventions, a number of soft-tissue fillers that can be injected subcutaneously or within the deeper layers of the skin have been developed or are under development.

Soft-tissue fillers are typically gels such as hydrogels. The practitioner using such soft-tissue filler, in particular dermal fillers, typically desires that such fillers do not provoke toxic or immunologic adverse effects when administered under the conditions of interest, show good biocompatibility, are injectable without burden, and base on natural materials. Concomitantly, fillers should remain in a spatially defined area and have a sufficient stability in biological systems such as when being injected.

A material used for soft-tissue filling in the art is hyaluronic acid (HA). Hyaluronic acid as such has been described as a potential filler as described in WO 2017/162676. A cross-linked hyaluronic acid hydrogel is also described in WO 2020/127407. Hyaluronic acid has a good biological acceptability. However, one significant drawback is that biodegradation of such material based on hyaluronic acid is comparably rapid and the filler material is not suitable for long-term solutions. It has limited longevity in subjects administered therewith, often of less than a desired minimum range of several months. When hyaluronic acid is degraded rapidly in the body, the viscosity decreases undesirably rapidly and the filling effect is not sufficiently long lasting.

Furthermore, storability and shelf life of hyaluronic acid-based filers is often limited. Stored product comprising unmodified hyaluronic acid often tend to partly degrade. Then, viscosity decreases and such stored and thus, partly degraded product that is then administered has an even shorter longevity in an administered subject.

Thus, there is the need for provision of further filling materials that have improved biological stability. Pharmaceutical compositions comprising fibroin fragments and other ingredients such as hyaluronic acid are also described in WO 2020/247887. Such blends do not have properties as desired. For instance, stability is rather limited. The obtained materials do not show stability to a degree that is desired for a number of cosmetic and therapeutic uses. Therefore, attempts have been made to improve the material properties further by cross-linking silk fibroin with hyaluronic acid by means of synthetic cross-linkers.

Another molecular entity used in the context of filling is silk fibroin. This is an injectable material having a good biocompatibility. It has a rather good stability but poor filling properties. It was however found that pure fibroin does not bear optimal gelation properties. Thus, it was considered to improve the properties of dermal fillers by mixing silk fibroin with elastic proteins such as elastin (cf. US 8,288,347).

Attempts were made to improve the material properties by simple mixing silk fibroin with unbound hyaluronic acid. US 8,288,347 mentions admixing cross-linked hyaluronic acid with fibroin-containing mixtures. Herein, hyaluronic acid and fibroin are unbound.

It has been considered to conjugate hyaluronic acid with silk fibroin by means of linkers connecting the polymeric strands. US 2014/0315828 describes a process for cross-linking unmodified hyaluronic acid and fibroin via a multiepoxide or multiamine cross-linking agent. US 2014/0315828 focuses on the preparation of gels comprising small-sized particles. For instance, US 2014/0315828 teaches the multiamine linker hexamethylene diamine (HMDA) and, in particular, the epoxide-based linker butanediol diglycidyl ether (BDDE). Also US-A 2018/0055971 teaches using multiamine linkers such as lysine methyl ester HMDA as well as epoxid linkers such as BDDE for conjugation. WO 2020/132331 teaches a tissue filler comprising silk fibroin, hyaluronic acid, and polyethylene glycol (PEG), wherein cross-linking is obtained via a linker moiety.

It is taught that the linker can be, for instance, selected from the group consisting of a polyepoxy linker, a diepoxy linker, a polyepoxy -PEG, a diepoxy -PEG, a polyglycidyl-PEG, a diglycidyl-PEG, a poly acrylate PEG, a diacrylate PEG, 1,4-bis(2,3-epoxypropoxy)butane, 1,4-bisglycidyloxybutane, divinyl sulfone (DVS), 1,4-butanediol diglycidyl ether (BDDE), UV light, glutaraldehyde, l,2-bis(2,3-epoxypropoxy)ethylene (EGDGE), 1,2, 7, 8-di epoxy octane (DEO), biscarbodiimide (BCDI), pentaerythritol tetraglycidyl ether (PETGE), adipic dihydrazide (ADH), bis(sulfosuccinimidyl)suberate (BS), hexamethylene diamine (HMDA), l-(2,3-epoxypropyl)-2, 3 -epoxy cyclohexane, a carbodiimide, and any combinations thereof. WO 2015/149941 teaches linking hyaluronic acid with heparosane via a BDDE linker. KR-A 2020/0036664 teaches photoinduced cross-linking o hyaluronic acid and fibroin using methacryl groups and photoinitiator such as lithium arylphosphinate.

The obtained dimensionally stable hydrogels, which contain such linker structure, however, comprise undesirable interconnecting linker moieties that are often of non-natural origin.

Often, the linker moieties are chemically reactive by themselves such as epoxide-based linker butanediol diglycidyl ether (BDDE). Residuals of such non-reacted or half-reacted bivalent linkers can be harmful and limit usability of the materials. There are maximally administrable contents of such reactive linkers and, thus, for safety reasons also for filler materials prepared by using such.

For instance, one or even both epoxy groups can still be present and react with cells. This can be harmful. Maximum amount that is administrable is limited. When administered to a subject in need thereof and degraded in said subject, xenobiotic and undegradable or poorly degradable metabolites can be generated. This is generally not desired, in particular not in cosmetic and pharmaceutic uses. Thus, there is a desire to avoid such xenobiotic linker moieties.

Piluso et al. (European Polymer Journal, 2018, 100:77-85) describes sequential alkyne-azide cycloadditions for functionalized gelatin hydrogel formation. Piluso et al. teaches that gelatin can be functionalized with various moieties, preferably small molecules. Hydrogels may be formed. Piluso et al. teach that propiolic acid may be conjugated by means of a carbodiimide activating agent and subsequently a gel may be formed via a bivalent linker. Reactive linkers can thus be avoided. However, this process is rather complex and requires several laborious steps and toxic agents. CN-A 111440340 describes a rather complex multi-step process for obtaining a silk fibroin-sodium hyaluronate cross-linked network. The silk fibroin is partly digested by contacting it with a tyrosinase and hydrogen peroxide. Hyaluronate is reacted with a derivative of ethanesulfonic acid, N-hydroxysuccinimide and a carbodiimide activator. In a third step, the two already reacted solutions are combined with each other. Such step is comparably laborious. Furthermore, undesirable reactive ingredients such as N-hydroxysuccinimide are used and can have detrimental effects when residuals remain in the obtained hydrogels.

WO 2019/175036 provides a porous biomaterial for tissue regeneration. This application teaches reacting fibroin moieties with formyl hyaluronic acid. Thereby, a direct linkage between the fibroin moieties and hyaluronic acid is obtained without a linker moiety introduced in between. A disadvantage of this method is that the rather reactive formyl hyaluronic acid is used and that imine groups are formed which are rarely found in nature. The reaction is taught including a reaction using a freeze-drying and drying step.

In view of above, there is still an unmet need for an efficient method for preparing a bio-based material that requires only low procedural efforts, that avoids xenobiotic linker moieties introduced into the hydrogel and minimizes the residuals of toxic reagents. It is further desired to obtain amide bonds and to avoid the need of the intermediate formation of formyl groups. Particularly desirable is a method that provides injectable hydrogel (e.g., usable as super-volumizer) for facial and body re-shaping.

Surprisingly, it has been found that a bio-based material having beneficial properties can be obtained from a method comprising the reaction of a fibroin moiety comprising primary amino residues with polysaccharide moieties comprising carboxylic acid residues or salt thereof with an activating agent forming an activation, thereby forming amide bonds.

A first aspect of the present invention relates to a method for preparing a cross-linked material, said method comprising:
(i) contacting the following components with each other:
   (A) one or more fibroin moieties comprising primary amino residues or salts thereof,
   (B) one or more polysaccharide moieties comprising carboxylic acid residues or salts thereof,
   (C) one or more activating agents that effect reaction of carboxylic acid residues with amino residues thereby forming amide bonds, and
   (D) one or more solvents; and
(ii) allowing a reaction of at least some of the carboxylic acid residues with at least some of the primary amino residues to form amide bonds conjugating the one or more fibroin moieties covalently with the one or more polysaccharide moieties; and
(iii) optionally purifying the cross-linked material obtained from step (ii).

It has been found that such cross-linked material bears unexpectedly beneficial properties. It has been found that cross-linked material obtainable from the method of the present invention has a long-lasting stability. Viscosity is widely maintained over weeks, even when incubated at elevated temperatures. Likewise, also enzymatic degradability is desirably diminished. It was surprisingly found that xenobiotic linker structures as commonly used in the prior rat could be avoided. The cross-linked material of the present invention may essentially consist of amino acid moieties and polysaccharide moieties, which can also found in nature. In aqueous environment, the obtained cross-linked material may form a hydrogel.

It may also be used to mimic an extracellular matrix and may, thus, induce cell proliferation and/or cellular migration. It may be well used as a filler (e.g., dermal filer) which may be populated by cells. The cross-linked material of the present invention may have good shear-thinning properties. Preferably, it has thixotropic properties. Thus, it is preferably less viscous when stressed. Accordingly, it may be injected very well, while still being rather viscous in its target area (when e.g., administered in a subcutaneous area). Comparably low extrusion forces are required. Gels of high viscosity and low extrusion force are obtainable. It may optionally serve as a super-volumizer.

The process of the present invention can be conducted without undue burden with comparably low efforts. No lubrication phase is found.

The claimed method is particularly beneficial compared to procedures described in the prior art because it required only three educts (raw materials) in addition to a solvent, i.e., one or more fibroin moieties, one or more polysaccharide moieties, and one or more activating agents. Further components such as linkers are not required.

The omittance of xenobiotic linker structures such as, e.g., BDDE, may enable higher amounts of material administered to a subject, e.g., injected subdermally Furthermore, it may have longevity in a subject's body after administration.

The obtainable cross-linked material of the present invention may have a long shelf life and storability due to the avoidance of reactive groups. It is also thermally comparably stable.

As used in the context of the present invention, the term "fibroin moiety" may be understood in the broadest sense as any moiety of fibroin known in the art.

In a preferred embodiment, the one or more fibroin moieties have a weight average molecular weight of at least 1 kDa, of at least 5 kDa, of at least at least 10 kDa, of at least 100 kDa, or of at least 200 kDa or more. Preferably, the one or more fibroin moieties are each polymeric moieties or a complex of polymeric moieties of a total molecular weight (Mw) of at least 5 kDa (5000 Dalton, 5 Kilodalton), more preferably at least at least 10 kDa (10000 Dalton), even more preferably at least 100 kDa, in particular at least 200 kDa or more. In one embodiment, the one or more fibroin moieties have a weight average molecular weight of 10 to 400 kDa.

In a preferred embodiment, the one or more fibroin moieties have a weight average not more than 2000 kDa, not more than 1000 kDa, not more than 750 kDa, not more than 500 kDa, not more than 250 kDa, not more than 200 kDa, or not more than 150 kDa.

In a preferred embodiment, the one or more fibroin moieties have a weight average molecular weight of at least 5 kDa, in the range of 5 to 1000 kDa, in the range of 5 to 400 kDa, in the range of 10 to 400 kDa, or in the range of 100 to 150 kDa. In a preferred embodiment, the one or more fibroin moieties have a weight average molecular weight of in the range of 10 to 400 kDa. In another preferred embodiment, the one or more fibroin moieties have a weight average molecular weight of in the range of 100 to 150 kDa.

In a particularly preferred embodiment, at least one of the one or more fibroin moieties, in particular all of the one or more fibroin moieties, may have a weight average molecular weight of 50 to 400 kDa. For instance, the one or more fibroin moieties may have a weight average molecular weight of 10 to 100 kDa, of 50 to 150 kDa, of 100 to 150 kDa, of 75 to 200 kDa, of 100 to 250 kDa, or of 200 to 400 kDa.

In a preferred embodiment, the fibroin moieties have at least two different weight average molecular weights each comprising primary amino residues or salts thereof. In other words, the fibroin moieties may also be a mixture of fibroin moieties of different weight average molecular weight. In a preferred embodiment, the fibroin moieties have at least two different molecular weights and at least one fibroin moiety has, preferably at least two fibroin moieties both have, in particular all fibroin moieties each have, a molecular weight in the range of 5 to 1000 kDa, in the range of 5 to 400 kDa, in the range of 10 to 400 kDa, in the range of 100 to 150 kDa, in the range of 10 to 100 kDa, in the range of 50 to 150 kDa, in the range of 100 to 150 kDa, in the range of 75 to 200 kDa, in the range of 100 to 250 kDa, or in the range of 200 to 400 kDa. In a preferred embodiment, the fibroin moieties have at least two different molecular weights and at least one fibroin moiety has, preferably at least two fibroin moieties both have, in particular all fibroin moieties each have, a molecular weight in the range of 50 to 400 kDa.

As used throughout the present invention, the molecular weight (Mw) is preferably the weight average molecular weight of the characterized species. Each fibroin moiety may have one or more backbones (amide/protein backbones) of one or more full-length fibroin polypeptides and/or one or more fibroin polypeptides or a complex of two or more thereof.

Preferably, a fibroin moiety comprises at least one backbone of a full-length fibroin polypeptide, in particular (essentially) consists of one or more backbones of one or more full-length fibroin polypeptides. In other words, a fibroin moiety is preferably derived from naturally occurring fibroin.

As used herein, amide binds are understandable in the broadest sense. Typically, an amide bond has the structure -NH-CO- or a tautomeric structure thereof. The amide bond formed between the fibroin moieties and the polysaccharide moieties may have any chirality. In one embodiment, it is a racemic mixture.

In a preferred embodiment, the one or more fibroin moieties are silk fibroin moieties, more preferably silk fibroin moieties having at least 80% sequence homology to a natural insect or spider silk fibroin moiety. In a preferred embodiment, fibroin is silk fibroin. In an alternative preferred embodiment, fibroin is a polypeptide or a complex of two or more polypeptides having at least 80 %, more preferably at least 90 %, even more preferably at least 95 %, even more preferably at least 98 % sequence homology, in particular identity, to one or more naturally occurring silk fibroin polypeptides. Silk fibroin may also include a truncated form thereof. Silk fibroin may be silkworm (*Bombyx mori*) fibroin and insect or spider silk fibroin.

The term "moiety" in the context of the t invention may be understood in the broadest sense as any molecular structure.

A moiety may be either a compound comprising or consisting of the respective structure or may form part of a larger chemical entity such as, e.g., the cross-linked material of the present invention. For instance, a fibroin moiety may be a fibroin or a chemical entity comprising fibroin. A fibroin moiety may optionally comprise more than one fibroin backbone conjugated with each other. Optionally, one or more fibroin backbones may bind to one or more other structures such as, in particular, the one or more polysaccharide moieties. It will be understood that the term "fibroin moiety" may also include salts and modified forms thereof.

According to the present invention, at least a part of the one or more fibroin moieties comprises primary amino residues or salts thereof. Amino groups can, for instance, form part of lysyl residues of the one or more fibroin moieties. Preferably, at least a part of the one or more fibroin moieties comprises one or more lysyl residues that may optionally be bound to the polysaccharide moiety.

As used in the context of the present invention, the term "fibroin" may be understood the broadest sense as any fibroin known in the art. Fibroin may be obtained from a commercial supplier (e.g., Advanced BioMatrix, USA (e.g. product No. 5154-20ML); CareSilk, Italy (e.g. product No. CSK10-1051)) or may be prepared from a natural source or by means of genetic engineering (also: bio-fermatation, biotechnological means) or rather synthetic engineering. For example, it may be fibroin of *Bombyx mori* or, alternatively, a species selected from the group consisting of *Antheraea*, *Cricula*, *Sami, Gonometa an Nephila* (e.g., *Nephila clavipes*) species or a homologue of having at least 80 %, more preferably at least 90 %, even more preferably at least 95 %, even more preferably at least 98 % sequence homology, in particular identity, to one of the aforementioned or a truncated form thereof. It will be understood that also mixtures of different fibroins may be used.

In a preferred embodiment, fibroin is ((essentially) complete) silkworm (*Bombyx mori*) fibroin. In a particularly preferred embodiment, fibroin is silkworm fibroin obtainable or obtained from *Bombyx mori*. Silkworm fibroin may be obtained from silkworm cocoons. The process of obtaining silk from silkworm is well-known in the art. For example, silkworm cocoons may be boiled for about 30 min (minutes) in an aqueous solution. Optionally, the aqueous solution may comprise about 0.02 M Na₂CO₃. The cocoons may be rinsed with water or an aqueous buffer to extract the sericin proteins and the extracted fibroin may be dissolved in an aqueous buffer. Salts that may be used for this purpose may, exemplarily, include lithium bromide, lithium thiocyanate, calcium nitrate and mixtures thereof. Optionally, the extracted fibroin may be dissolved in about 9-12 M lithium bromide solution.

The salt may be removed by any means, e.g., dialysis. In a preferred embodiment, other components of the silkworm cocoons have been (essentially) removed such as, e.g., sericin. Thus, preferably, at least 50 wt.%, in more preferably at least 75 wt.%, even more preferably at least 80 wt.%, particular at least 90 wt.%, of the sericin initially contained in the silkworm cocoon have been removed. Silk fibroin may be type I, type II or type III silk fibroin or a mixture of two or more thereof. Preferably, fibroin is or comprises type I silk fibroin. Fibroin may bear the properties as described in the art such as described in US 2014/315828.

Alternatively, one or more fibroin polypeptides, including silkworm fibroin polypeptides and complete silkworm fibroin, may also be obtained by means of genetic engineering. Genetically engineered fibroin may be, for example, obtained from bacteria, insect cells, spider cells, yeast, mammalian cells, transgenic animals, or transgenic plants.

The one or more fibroin moieties may be stored at any condition. For instance, the one or more fibroin moieties may be stored in a freezer or a liquid gas, e.g. in a temperature range of from -15°C to -200°C. For example, the one or more fibroin moieties may be stored at approximately -80°C or in liquid nitrogen (i.e., at approximately -196°C). The one or more fibroin moieties may be stored in dry state as a powder or as a solution in water (e.g., in a concentration in the range of from 10 to 100 mg/ml (e.g., approximately 50 mg/ml). When thawing a previously frozen solid fibroin or fibroin solution, preferably, the one or more fibroin moieties may be optionally protected from air.

As used in the context of the present invention, the term "polysaccharide moiety" may be understood the broadest sense as any moiety of a polysaccharide known in the art which comprises carboxylic acid residues or salts thereof. As used in the context of the present invention, the term "polysaccharide" may be understood the broadest sense as any polysaccharide in the art. According to the present invention, at least one polysaccharide moiety comprises at least one carboxylic acid residue or salt thereof. Preferably, the one or more polysaccharide moieties further comprises hydroxy groups. A polysaccharide may a naturally occurring polysaccharide that may be modified or may be a synthetic polysaccharide. In this context, a polysaccharide may be branched or unbranched. It will be understood that the term "polysaccharide moiety" may also include salts and modified forms thereof. In a preferred embodiment, the polysaccharide has not been oxidized.

Preferably, at least one polysaccharide moiety is a polymeric moiety of a weight average molecular weight (Mw) of at least 1 kDa (1000 Da), more preferably at least 5 kDa, even more preferably at least 10 kDa, even more preferably at least 50 kDa, even more preferably at least 100 kDa, even more preferably at least 200 kDa, even more preferably at least 300 kDa or more. Preferably, the one or more polysaccharide moieties have a weight average molecular weight (Mw) in the range of from 10 to 10000 kDa. In a preferred embodiment, the one or more polysaccharide moieties have a weight average molecular weight of at least 50 kDa, in particular in the range of 50 to 4000 kDa. More preferably, the one or more polysaccharide moieties have a weight average Mw in the range of from 100 to 10000 kDa. In one embodiment, the at least one polysaccharide moiety is a polymeric moiety having an intrinsic viscosity of 1.0 to 3.3 m³/kg (20°C, 1013 hPa, water).

In a particularly preferred embodiment, at least one of the one or more polysaccharide moieties, in particular all of the one or more polysaccharide moieties, may have a weight average molecular weight of 1500 to 3500 kDa (1.5 and 3.5 MDa). More preferably, it may have a weight average molecular weight in the range of from 100 and 5000 kDa, of from 200 to 2000 kDa, of from 250 to 1500 kDa, of from 300 to 1000 kDa, of from 400 to 900 kDa or of from 500 to 900 kDa.

In a preferred embodiment, the one or more polysaccharide moieties comprise one or more types of sugar acid moieties or salts thereof.

In a preferred embodiment, the one or more polysaccharide moieties comprise one or more types of sugar acid moieties or salts thereof, wherein the one or more types of sugar acid moieties are selected from the group consisting of:
(B1) one or more uronic acid moieties, in particular selected from the group consisting of glucuronic acid moiety, galacturonic acid moiety, iduronic acid moiety, and combinations of two or more thereof;
(B2) one or more aldonic acid moieties, in particular selected from the group consisting of glyceric acid moiety, xylonic acid moiety, gluconic acid moiety, ascorbic acid moiety, and combinations of two or more thereof;
(B3) one or more ulosonic acid moieties, in particular selected from the group consisting of neuraminic acid moiety, ketodeoxyoctulosonic acid moiety, and combinations thereof; and/or
(B4) one or more aldaric acid moieties, in particular selected from the group consisting of tartaric acid moiety, meso-galactaric acid moiety, glucaric acid moiety, and combinations of two or more thereof.

In a preferred embodiment, the one or more polysaccharide moieties comprise uronic acid moieties. In a preferred embodiment, the one or more polysaccharide moieties comprise glucuronic acid moieties. In a preferred embodiment, the one or more polysaccharide moieties comprise D-glucuronic acid moieties.

In a preferred embodiment, the one or more polysaccharide moieties comprise or consist of D-sugar moieties. In an alternative embodiment, the one or more polysaccharide moieties comprise or consist of L-sugar moieties. In an alternative embodiment, the one or more polysaccharide moieties comprise or consist of a combination of D- sugar moieties and L-sugar moieties. For instance, in such combination, racemic mixture of sugar moieties may be comprised or specific sugar moieties are D-sugar moieties and others are L-sugar moieties. .

In a preferred embodiment, the one or more polysaccharide moieties comprise or consist of one or more glycosaminoglycan moieties. In a preferred embodiment, the one or more polysaccharide moieties are selected from the group consisting of hyaluronic acid (HA) moieties, heparosan moieties, heparin, chondroitin sulphate, and mixtures of two or more thereof. In a preferred embodiment, the one or more polysaccharide moieties comprise or consist of hyaluronic acid, heparosan, chondroitin sulfate, and carboxymethyl cellulose. Such polysaccharides comprising carboxylic acid groups are also commercially available (e.g., from HTL Biotechnology, Javene, France).

In a preferred embodiment, the one or more polysaccharide moieties comprise or consist of one or more hyaluronic acid moieties.

In a preferred embodiment, the cross-linked material of the present invention is a gel. In a preferred embodiment, the cross-linked material of the present invention is a polysaccharide/fibroin gel. In a preferred embodiment, the cross-linked material of the present invention is a hyaluronic acid/fibroin gel (HA/fibroin gel).

Hyaluronic acid (also: HA, hyaluronate, or hyaluronan) may be understood in the broadest sense as any hyaluronic acid in the art. It may be polysaacharide moiety that contains hyaluronic acid moieties (also hyaluronic acid units), preferably comprises at least 50 mol% of hyaluronic acid moieties, more preferably at least 75 mol%, even more preferably at least 80 mol%, even more preferably at least 90 mol%, referred to the whole content of saccharide moieties in the polysaccharide, of hyaluronic acid moieties. Hyaluronic acid may optionally comprise one or more saccharide moieties other than hyaluronic acid. Hyaluronic acid may optionally be partly modified. It may, for instance, be partly oxidized and may bear aldehyde groups and/or may be cross-linked. Such modifications are described, for instance in WO 2020/127407.

In a preferred embodiment, hyaluronic acid is a naturally glycosaminoglycan composed of linked repeating units of V-acetyl-D-glucosamine and D-glucuronic acid ([alpha-1,4-D-glucuronic acid-beta-1,3-N-acetyl-D-glucosamine]ₙ). Accordingly, the repeating unit of hyaluronic acid may be exemplarity the following:

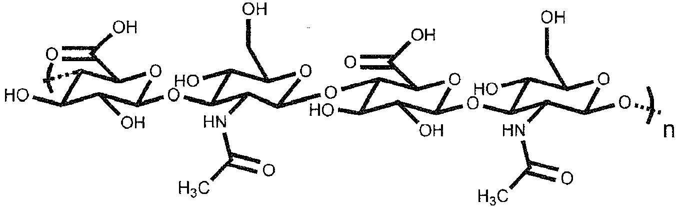

Hyaluronic acid may be used as described in WO 2017/162676. Also cross-linked and optionally modified hyaluronic acid such as described in WO 2020/127407 may be used as hyaluronic acid in the context of the present invention.

The weight average molecular weight (Mw) of hyaluronic acid in the context of the present invention is preferably at least 1 kDa (1000 Da), more preferably at least 5 kDa, even more preferably at least 10 kDa, even more preferably at least 50 kDa, even more preferably at least 100 kDa, even more preferably at least 200 kDa, even more preferably at least 300 kDa or more. The weight average molecular weight (Mw) of hyaluronic acid in the context of the present invention is preferably in the range of from 10 to 10000 kDa, more preferably 100 to 10000 kDa, or 100 to 5000 kDa. In a more preferred embodiment, hyaluronic acid has a weight average molecular weight (Mw) in the range of from 50 to 4000 kDa. More preferably, hyaluronic acid has a weight average Mw in the range of from 100 to 3500 kDa, of from 200 to 2000 kDa, of from 250 to 1500 kDa, of from 300 to 1000 kDa, of from 400 to 900 kDa or of from 500 to 900 kDa.

In a particularly preferred embodiment, at least one of the one or more polysaccharide moieties are one or more hyaluronic acid moieties, wherein at least one of the one or more hyaluronic acid moieties, in particular all of the one or more hyaluronic acid, may have a weight average molecular weight of 1500 to 3500 kDa.

Heparosan may be understood in the broadest sense as any heparosan. In a preferred embodiment, it may be such as described in WO 2015/149941. Heparosan (HEP) is a biopolymer belonging to the glycosaminoglycan (GAG) family of polysaccharides.

In humans, it is an intermediate product in the biosynthesis of heparin and heparin sulfate. The structure of heparosan is highly similar to that of hyaluronic acid (HA) since it has the same monosaccharide component sugars as hyaluronic acid and differs from HA only in that the beta-(1,3) glycosidic bond between the glucuronic acid (GlcUA) and the *N*-acetylglucosamine (GlcNAc) in HA is replaced by a beta-(1,4) glycosidic bond in HEP and in that the beta-(1,4) glycosidic bond between *N-*acetylglucosamine (GlcNAc) and the glucuronic acid (GlcUA) in HA is replaced by an alpha-(1,4) glycosidic bond in HEP:
GlcUA-beta-(1-4)-[GlcNAc-alpha-(1-4)-GlcUA-beta-(1-4)]ₙ-GlcNAc HEP

Typically, heparosan has excellent biocompatibility. Heparosan carries a high number of negative charges and hydroxyl groups and is therefore highly hydrophilic, which increases tissue compatibility. Furthermore, due to the fact that heparosan polymers, even after modification, still comprise stretches that occur in natural heparan sulfate and heparin polymers, heparosan is typically non-immunogenic (e.g., does not induce antibodies). Moreover, due to the structural similarity between heparosan and hyaluronic acid, the same chemical modifications, including oxidation to aldehydes as that known for hyaluronic acid may be made on the functional groups. The molecular weight (Mw) of the heparosan polymers used in the context of the present invention may have any molecular weight.

In a preferred embodiment, the polysaccharide moieties (component b), in particular hyaluronic acid moieties, have at least two different molecular weights each comprising primary amino residues or salts thereof. In other words, the polysaccharide moieties may also be a mixture of polysaccharide moieties of different molecular weight. In a preferred embodiment, the polysaccharide moieties have at least two different molecular weights and at least one polysaccharide moiety has, preferably at least two polysaccharide moieties, in particular all polysaccharide moieties, each have a molecular weight in the range of 10 to 10000 kDa, in the range of 100 to 10000 kDa, or in the range of 100 to 5000 kDa. in the range of from 100 to 3500 kDa, in the range of from 200 to 2000 kDa, in the range of from 250 to 1500 kDa, in the range of from 300 to 1000 kDa, in the range of from 400 to 900 kDa, or in the range of from 500 to 900 kDa.

In a preferred embodiment, the polysaccharide moieties have at least two different molecular weights and at least one polysaccharide moiety has, preferably at least two polysaccharide moieties, in particular all polysaccharide moieties, each have a molecular weight in the range of 1500 to 3500 kDa.

In a preferred embodiment, the polysaccharide moieties comprise or consist of at least two hyaluronic acid moieties having at least two different molecular weights and at least one hyaluronic acid moiety has, preferably at least two hyaluronic acid moieties both have, in particular all hyaluronic acid moieties each have, a molecular weight in the range of 10 to 10000 kDa, in the range of 100 to 10000 kDa, or in the range of 100 to 5000 kDa. in the range of from 100 to 3500 kDa, in the range of from 200 to 2000 kDa, in the range of from 250 to 1500 kDa, in the range of from 300 to 1000 kDa, in the range of from 400 to 900 kDa, or in the range of from 500 to 900 kDa. In a preferred embodiment, the polysaccharide moieties comprise or consist of at least two hyaluronic acid moieties having at least two different molecular weights and at least one hyaluronic acid moiety has, preferably at least two hyaluronic acid moieties both have, in particular all hyaluronic acid moieties each have, a molecular weight in the range of 1500 to 3500 kDa.

The (mass) ratio between the total mass of the one or more fibroin moieties (component A) and the total mass of the one or more polysaccharide moieties (component B) may be any ratio. When particularly high water/buffer absorbance is desired, the polysaccharide moieties may be used in mass excess. When particularly high stability is desired, the fibroin moieties may be used in larger mass excess. Preferably in the (mass) ratio A:B in the range of from 1:100 to 100:1. 1.

In a preferred embodiment, the mass ratio between the one or more fibroin moieties (A) and the one or more polysaccharide moieties (B) A:B is in the range of 5:1 to 1:20, preferably in the range of 1:1 to 1:10, in particular in the range of 1:1 to 1:5.

For instance, the mass ratio between the one or more fibroin moieties (A) and the one or more polysaccharide moieties (B) A:B may be in the range of 1:9 to 2:1, of 1:8 to 1.5:1, of 1:7 to 1:1, of 1:6 to 1:1, of 1:5 to 1:1, of 1:4 to 1:1, of 1:3 to 1:1, of 1:2 to 1:1, or of 1:1.5 to 1:1.

In a preferred embodiment, the fibroin moieties have at least two different molecular weights each comprising primary amino residues or salts thereof, and the polysaccharide moieties, in particular hyaluronic acid moieties, have at least two different molecular weights each comprising primary amino residues or salts thereof.

In a preferred embodiment:
(a) the fibroin moieties have at least two different molecular weights and at least one fibroin moiety has, preferably at least two fibroin moieties both have, in particular all fibroin moieties each have, a molecular weight in the range of 5 to 1000 kDa, in the range of 5 to 400 kDa, in the range of 10 to 400 kDa, in the range of 100 to 150 kDa, in the range of 10 to 100 kDa, in the range of 50 to 150 kDa, in the range of 100 to 150 kDa, in the range of 75 to 200 kDa, in the range of 100 to 250 kDa, or in the range of 200 to 400 kDa; and
(b) the hyaluronic acid moieties have at least two different molecular weights and at least one hyaluronic acid moiety has, preferably at least two hyaluronic acid moieties both have, in particular all hyaluronic acid moieties each have, a molecular weight in the range of 10 to 10000 kDa, in the range of 100 to 10000 kDa, or in the range of 100 to 5000 kDa. in the range of from 100 to 3500 kDa, in the range of from 200 to 2000 kDa, in the range of from 250 to 1500 kDa, in the range of from 300 to 1000 kDa, in the range of from 400 to 900 kDa, or in the range of from 500 to 900 kDa.

In a preferred embodiment:
(a) the fibroin moieties have at least two different molecular weights and at least one fibroin moiety has, preferably at least two fibroin moieties both have, in particular all fibroin moieties each have, a molecular weight in the range of 5 to 1000 kDa, in the range of 50 to 400 kDa; and
(b) the hyaluronic acid moieties have at least two different molecular weights and at least one hyaluronic acid moiety has, preferably at least two hyaluronic acid moieties both have, in particular all hyaluronic acid moieties each have, a molecular weight in the range of 1500 to 3500 kDa.

As used in the context of the present invention, an activating agent may be any compound that effects reaction of carboxylic acid residues with amino residues thereby forming amide bonds. It will be understood that it is mainly meant that an activating agent is a compound that effects reaction of carboxylic acid residues of the one or more polysaccharide moieties with amino residues of the one or more fibroin moieties thereby forming amide bonds.

In a preferred embodiment, the one or more activating agents are selected from the group consisting of
(C1) one or more triazine-based activating agents, in particular selected from the group consisting of 4-(4,6-dimethoxy-1,3,5-triazin-2-yl)-4-methylmorpholinium (DMTMM), a salt thereof, and/or 2-chloro-4,6,-dimethoxy-1,3,5-triazine (CDMT) and combinations thereof;
(C2) one or more carbodiimide activating agents, in particular selected from the group consisting of N,N'-dicyclohexylcarbodiimide (DCC), diisopropylcarbodiimide (DIC), 1-ethyl-3-(3-dimethylaminopropyl)carbodiimid (EDC), and combinations of two or more thereof; and
(C3) combinations thereof.

According to the present invention, an activating agent is typically not covalently included in the cross-linked material. Thus, it can be typically optionally removed from the cross-linked material of the present invention by any means such as, e.g., washing, filtration, etc.

In a preferred embodiment, a triazine-based activating agent is 4-(4,6-dimethoxy-1,3,5-triazin-2-yl)-4-methylmorpholinium (DMTMM) or a salt thereof, preferably it is a 4-(4,6-dimethoxy-1,3,5-triazin-2-yl)-4-methylmorpholinium salt, A salt of DMTMM is preferably a salt wherein the counter-ion is an anion that is cosmetically and/or pharmaceutically acceptable such as, e .g., chloride, acetate, bicarbonate (hydrogen carbonate), or a mixture of two or more anions.

In a preferred embodiment, a triazine-based activating agent is 4-(4,6-dimethoxy-1,3,5-triazin-2-yl)-4-methylmorpholinium chloride.

In a preferred embodiment, a carbodiimide activating agent is N,N'-dicyclohexylcarbodiimide (DCC), or 1-ethyl-3-(3-dimethylaminopropyl)carbodiimid (EDC).

In a preferred embodiment, the activating agent is 4-(4,6-dimethoxy-1,3,5-triazin-2-yl)-4-methylmorpholinium (DMTMM) or a salt thereof. Preferably, it is a 4-(4,6-dimethoxy-1,3,5-triazin-2-yl)-4-methylmorpholinium salt, in particular 4-(4,6-dimethoxy-1,3,5-triazin-2-yl)-4-methylmorpholinium chloride (CAS No. 3945-69-5).

DMTMM is considered as having a comparably low and essentially negligible toxicity, is not cancerogenic, not mutagenic, and not teratogenic/reprotoxic in the generally used amounts. Thus, it is particularly well usable for preparing a soft-tissue filler such as a dermal or connective tissue filler.

When using DMTMM as activating agent, 4-methylmorpholine (NMM) and/or 4,6-dimethoxy-1,3-5-triazine-2-ol (DMT) may be formed as degradation product(s).

In a preferred embodiment, the method is further characterized in that it comprises a step (iii) of purifying the cross-linked material by filtration, washing and/or dialysis, in particular crossflow filtration, diafiltration and/or dead-end filtration.

It will be understood that filtration may be crossflow filtration, dead-end filtration, or a combination of both. Filtration may be performed by any means. In the context of filtration, the a filter may have any pore size suitable for purifying the cross-linked material, i.e., preferably withholding the cross-linked material and allowing the passage of reactants and optionally of non-reacted polysaccharides and/or fibroin. Optionally, the pore size may be in a range of 5 nm to 2 µm, more particularly a pore size of 30 nm to 600 nm, more particularly a pore size of 80 nm to 300 nm, particularly a pore size of 5 nm to 60 nm. A filter may be of any material such as, e.g., ceramic, metal, polymer material, or a combination thereof.

Optionally, filtration may be dynamic filtration such as, e.g., described in WO 2020/030629. Accordingly, step (iii) may optionally comprise dynamic filtration of the cross-linked material, optionally, comprising the following steps:
a) transferring the cross-linked material in a dynamic filtration device which is equipped with semipermeable filter disc(s) and diafiltrating the gel comprising the steps of:
   i) concentrating the cross-linked material by applying a rotational speed within the range of 20 1/min to 500 1/min and a overpressure within the range of 0.5 to 6 bar to a predetermined concentration; or pumping the cross-linked material directly into the process chamber of the dynamic filtration device;
   ii) conducting a diafiltration to reduce unwanted molecules by applying a rotational speed within the range of 20 1/min to 500 1/min and a overpressure within the range of 0.5 to 6 bar;
b) optionally adding a mixture comprising a non-cross-linked material and water to the cross-linked material.

In one embodiment, the dynamic filtration device is equipped with 1 to 10 semipermeable filter disc(s). In DCF, any rotational speed and pressure may be used, such as, e.g., a rotational speed within the range of 20 1/min to 500 1/min and a pressure within the range of 0.5 to 3 bar. In DCF, any concentration may be used such as e.g., 10 to 70 mg/g.

Optionally, one or more further components (e.g., one or more local anesthetics (e.g., as laid out below such as, e.g., lidocaine), one or more cell proliferation factors, one or more dyes, and combinations of two or more thereof) may be added before, during or after conducting step (iii) of purifying the cross-linked material by filtration, washing and/or dialysis.

Step (iii) may be conducted for any time suitable for this purpose. Optionally, step (ii) may be conducted for 1 min to 1 week or longer, 2 min to 5 days, 3 min to 4 days, 5 min to 72 hours, 5 min to 24 hours, 10 min to 12 hours, 30 min to 6 hours, 1 hour to 5 hours, or 2 to 4 hours.

Step (iii) may be conducted for at any temperature suitable for this purpose such as, e.g., at 0°C to 100°C, at 4°C to 95°C, at 10°C to 70°C, at 15°C to 30°C, at 18 to 25°C, at 20°C to 70°C, at 20°C to 40°C, or at 60°C to 70°C.

In an embodiment of the present invention, crossflow filtration (also: cross-flow filtration) is dynamic crossflow filtration (DCF). Thus, in an embodiment, the method may be further characterized in that it comprises a step (iii) of purifying the cross-linked material by DCF. This is exemplified below. Optionally, DCF may be such as described in WO 2020/030629.

In a preferred embodiment, the method is further characterized in that steps (i) and (ii) are conducted in a single batch.

In a preferred embodiment, the method is further characterized in that step (i) is conducted at a temperature in the range of from 5 to 90°C, in particular 18 to 30°C,more in particular at ambient conditions (e.g., often 18 to 25°C). In a preferred embodiment, the method is further characterized in that step (ii) is conducted at a temperature in the range of from 5 to 90°C, in particular 18 to 30°C ,more in particular at ambient conditions (e.g., often 18 to 25°C). In a preferred embodiment, the method is further characterized in that step (iii), at far as present, is conducted at a temperature in the range of from 5 to 90°C, in particular 18 to 30°C, more in particular at ambient conditions (e.g., often 18 to 25°C).

In a preferred embodiment, the method is further characterized in that steps (i) and (ii) and optional step (iii) are conducted at a temperature in the range of from 5 to 90°C, in particular 18 to 30°C. In a preferred embodiment, these steps are conducted at ambient conditions (e.g., often 18 to 25°C). For instance, steps (i) and/or (ii) and/or l step (iii) may be conducted at a temperature of approximately 18°C, of approximately 19°C, of approximately 20°C, of approximately 21°C, of approximately 22°C, of approximately 23°C, of approximately 24°C, of approximately 25°C, of approximately 26°C, of approximately 27°C, of approximately 28°C, of approximately 29°C, or of approximately 30°C.

Steps (i) and (ii) and optional step (iii) may be conducted at any pressure. For example, pressure may be ambient pressure (e.g, often approximately 970 to 1100 hPa outer pressure).

Step (i) of contacting the components with each other may be conducted by any means. In a preferred embodiment, the method is further characterized in that step (i) involves the mixing of the components, i.e., the one or more fibroin moieties (as component A), the one or more polysaccharide moieties (as component B), the one or more activating agents (as component C), and one or more solvents (as component D), and optional one or more further components. Such mixing may be conducted by any means such as, e.g., by means of stirring and/or shaking.

In a preferred embodiment, the one or more polysaccharide moieties (as component B) and the one or more activating agents (as component C) are dissolved in one or more solvents (as component D) in a first step without the one or more fibroin moieties (as component A) and incubated.

This may activate the carboxylic groups of the polysaccharide moieties. Incubation may be conducted for any time sufficient for such purpose. In a preferred embodiment, in a first sub-step, the one or more polysaccharide moieties are dissolved in the one or more solvents and in a subsequent sub-step, the one or more activating agents are added, together representing the activation step.

Exemplarity, such activation step, may be performed for 5 min to 24 hours, for 10 min to 12 hours, for 30 min to 6 hours, for 45 min to 5 hours, for 45 min to 4 hours, or for 1 to 3 hours. Exemplarity, such activation step, may be conducted at any temperature such as, e.g., at 0°C to 100°C, at 4°C to 95°C, at 10°C to 70°C, at 15°C to 30°C, at 18 to 25°C, at 20°C to 70°C, at 20°C to 40°C, or at 60°C to 70°C, in particular at ambient temperature (e.g., often 18 to 25°C). For instance, incubation may be 1 min to 24 hours, in particular 30 min to 2 hours or 1 to 3 hours, at a temperature of 10 to 25°C, in particular 18 to 22°C. After such incubation, the one or more fibroin moieties (as component A) may be added. This may be further incubated to conduct step (ii) of the method of the present invention. Step (ii) may be conducted using any suitable solvent such as water or an aqueous buffer. Optionally, the solution may be stirred during the reaction step.

Accordingly, in a preferred embodiment, step (i) of the method comprises the following sub-steps:
(ia) contacting the following components with each other:
   (B) one or more polysaccharide moieties comprising carboxylic acid residues or salts thereof,
   (C) one or more activating agents that effect reaction of carboxylic acid residues with amino residues thereby forming amide bonds, and
   (D) one or more solvents,
   preferably wherein the one or more polysaccharide moieties (B) are first dissolved in the one or more solvents (D) and subsequently the one or more activating agents (C) are added;
(ib) allowing a reaction of at least some of the carboxylic acid residues with one or more activating agents thereby forming one or more activated polysaccharide moieties (also: polysaccharide-activating agent conjugates);
   and
(ic) adding to the activated polysaccharide moieties of sub-step (ib):
   (A) one or more fibroin moieties comprising primary amino residues or salts thereof.

In another preferred embodiment, the components A, B, C and D and optionally one or more further components are all mixed at once.

Step (ii) may be conducted for any suitable reaction time. For example, reaction time may be in the range of 1 min to 7 days, preferably of 5 min and 2 days, more preferably of 10 min and 24 hours. For example, reaction time may be in the range of 15 min to 24 hours, or of 30 min to 12 hours, or of 45 min to 6 hours, or of 1 hour to 4 hours.

In a preferred embodiment, the method comprises:
(i) contacting the following components with each other:
   (A) one or more silk fibroin moieties having a weight average molecular weight of at least 5 kDa that comprises primary amino residues or salts thereof,
   (B) one or more hyaluronic acid moieties having a weight average molecular weight of at least 50 kDa that comprises carboxylic acid residues or salts thereof,
   (C) one or more triazine-based activating agents that effect reaction of carboxylic acid residues with amino residues thereby forming amide bonds, in particular wherein the activating agent is 4-(4,6-dimethoxy-1,3,5-triazin-2-yl)-4-methylmorpholinium or a salt thereof; and
   (D) one or more solvents; and
(ii) allowing a reaction of at least some of the carboxylic acid residues with at least some of the primary amino residues to form amide bonds conjugating the one or more silk fibroin moieties covalently with the one or more hyaluronic acid moieties; and
(iii) optionally purifying the cross-linked material obtained from step (ii).

Any solvent usable as component D for the method of the present invention may be used. In a preferred embodiment, a polar solvent is used. In a preferred embodiment, a protic solvent is used. In a preferred embodiment, a protic polar solvent is used.

In a preferred embodiment, the solvent usable as component D comprises more than 50 wt.%, of at least 60 wt.%, of at least 70 wt.%, of at least 80 wt.%, of at least 90 wt.%, of at least 95 wt.%, or even 100 wt.%, referred to the total mass of the solvent, of one or more components selected from the group consisting of:
water;
one or more alcohols, preferably one or more C₁-C₅-alcohols, more preferably, one or more C₁-C₅-alcohols selected from the group consisting of methanol, ethanol, n-propanol, isopropanol, n-butanol (1-butanol), sec-butanol (2-butanol) isobutanol, (2-methylpropan-1-ol), tert-butanol (2-methylpropanol), pentan-1-ol, 2-methylbutan-1-ol, 3-methylbutan-1-ol, 2,2-dimethylpropan-1-ol, pentan-2-ol, 3-methylbutan-2-ol, pentan-3-ol, and/or 2-methylbutan-2-ol, and a combination of two or more thereof, in particular methanol and/or ethanol;
one or more primary amines, in particular in particular one or more C₁-C₅-amines; one or more carbonic acids, preferably one or more C₁-C₅-carbonic acids selected from the group consisting of formic acid, acetic acid, propionic acid, butyric acid, valerianic acid, isovalerianic acid, in particular formic acid and/or acetic acid;
one or more primary or secondary amides, preferably one or more C₁-C₅-amides, in particular formamide;
one or more sulfoxides, preferably one or more C₁-C₅-amides, in particular dimethyl sulfoxide (DMSO);
and a combination of two or more thereof.

In a preferred embodiment, an aqueous solvent is used, i.e., a solvent that comprises a water content by weight of more than 50 wt.%, of at least 60 wt.%, of at least 70 wt.%, of at least 80 wt.%, of at least 90 wt.%, of at least 95 wt.%, or even 100 wt.%, referred to the total mass of the solvent. In one embodiment of the present invention, an aqueous buffer comprises, in addition to water, one or more components selected from the group consisting of one or more alcohols (in particular one or more C₁-C₅-alcohols such as, e.g., methanol, ethanol, n-propanol, isopropanol, n-butanol (1-butanol), sec-butanol (2-butanol) isobutanol, (2-methyl-propan-1-ol), tert-butanol (2-methylpropanol), pentan-1-ol, 2-methylbutan-1-ol, 3-methylbutan-1-ol, 2,2-dimethylpropan-1-ol, pentan-2-ol, 3-methylbutan-2-ol, pentan-3-ol, and/or 2-methylbutan-2-ol), one or more primary amines (in particular one or more C₁-C₅-amines), one or more carbonic acids (in particular one or more C₁-C₅-carbonic acids such as., e.g., formic acid, acetic acid, propionic acid, butyric acid, valerianic acid, isovalerianic acid), one or more primary or secondary amides (in particular one or more C₁-C₅-amides such as, e.g., formamide), one or more sulfoxides (in particular one or more C₁-C₅-amides such as, e.g., dimethyl sulfoxide (DMSO)), one or more inorganic or organic cations (in particular one or more inorganic or organic cations of a molecular weight or less than 1000 Da, in particular alkali cations or earth alkali cations, other metal cations, protons, ammonium cations, etc.), one or more inorganic or organic anions (in particular one or more inorganic or organic anions of a molecular weight or less than 1000 Da, in particular chlorine, sulfate, etc.), one or more silicates, and a combination of two or more thereof.

In a preferred embodiment, water or an aqueous buffer (e.g., phosphate buffered saline (PBS), Tris buffer, borate buffer, acetic acid-buffered buffer, etc.) is used as solvent. In a preferred embodiment, a hydroalcoholic solvent is used such as, e.g., a mixture of water with ethanol, methanol, propanol, butanol, and/or pentanol. In a preferred embodiment, water is used as solvent D.

Water as used herein, may be understood in the broadest sense. Preferably, water is deionized water, distilled water, or tap water, in particular deionized water or distilled water.

As indicated above, in a preferred embodiment, step (i) of the method comprises the following sub-steps:
(ia) contacting the following components with each other:
   (B) one or more hyaluronic acid moieties having a weight average molecular weight of at least 50 kDa that comprises carboxylic acid residues or salts thereof,
   (C) one or more triazine-based activating agents that effect reaction of carboxylic acid residues with amino residues thereby forming amide bonds, in particular wherein the activating agent is 4-(4,6-dimethoxy-1,3,5-triazin-2-yl)-4-methylmorpholinium or a salt thereof;
   (D) one or more solvents,
   preferably wherein the one or more one or more hyaluronic acid moieties (B) are first dissolved in the one or more solvents (D) and subsequently the one or more triazine-based activating agents (C) are added;
(ib) allowing a reaction of at least some of the carboxylic acid residues with one or more triazine-based activating agents thereby forming one or more activated hyaluronic acid moieties (also: hyaluronic acid-activating agent conjugates); and
(ic) adding to the activated hyaluronic acid moieties of sub-step (ib):
   (A) one or more silk fibroin moieties having a weight average molecular weight of at least 5 kDa that comprises primary amino residues or salts thereof.

In an embodiment of the present invention, the method of the invention composes the following (preferably sequential) step:
dissolution of polysaccharide (preferably hyaluronic acid, in particular hyaluronic acid sodium salt) in water or buffer;
addition of activator (preferably DMTMM) to the polysaccharide solution;
allowing activation (preferably for some hours, e.g. at a temperature of 18 to 22°C); adding fibroin solution to the activated polysaccharide;
stirring and allowing the formation of the cross-linked material (preferably for some hours, e.g. at a temperature of 18 to 22°C);
purifying the cross-linked material (e.g. removal of DMTMM and degradation products thereof, e.g., by means of filtration and/or dialysis, e.g. at a temperature of 18 to 22°C);
optionally adding an anesthetic (e.g., lidocaine); and
optionally sterilizing.

A further aspect of the present invention relates to a cross-linked material obtainable from a method of the present invention.

It will be understood that the definitions and preferred embodiments as laid out in the context of the method of the present invention *mutatis mutandis* apply to the cross-linked material of the present invention.

In an embodiment of the present invention, the cross-linked material has been prepared by using DMTMM as activating agent. In an embodiment of the present invention, the cross-linked material, in particular before final purification, comprises DMTMM, 4-methylmorpholine (NMM), and/or 4,6-dimethoxy-1,3-5-triazine-2-ol (DMT). In an embodiment of the present invention, the cross-linked material, in particular before final purification, comprises NMM and/or DMT. In an embodiment of the present invention, the cross-linked material, in particular before final purification, comprises up to 0.1 % by weight, preferably 0.01 to 1000 ppm, 0.1 to 100 ppm, or 1 to 10 ppm of NMM and/or DMT, referred to the total weight of the cross-linked material (gel).

A further aspect of the present invention relates to a cross-linked material comprising or consisting of
(A) one or more fibroin moieties having a weight average molecular weight of at least 5 kDa, and
(B) one or more hyaluronic acid moieties having a weight average molecular weight of at least 50 kDa,
wherein the one or more fibroin moieties are covalently conjugated with the with the one or more hyaluronic acid moieties via amide bonds without an interconnecting linker structure.

As used herein, the term "without an interconnecting linker structure" may be understood in the broadest sense in that no further chemical moiety that does not originate from (also: is not present in) fibroin moieties or polysaccharide moieties (e.g., hyaluronic acid moieties) is introduced into the chemical structure that conjugates one or more fibroin moieties covalently with one or more hyaluronic acid moieties via amide bonds. In other words, the amide bonds are preferably formed from inclusion of a nitrogen atom originating from fibroin (e.g., a lysinyl side chain) and from inclusion of a carbon atom originating from polysaccharide moieties (e.g., hyaluronic acid moieties).

In a preferred embodiment, the cross-linked material is further characterized in that it does not comprise imide groups. In a preferred embodiment, the cross-linked material is further characterized in that it does not comprise imine groups. In a preferred embodiment, the cross-linked material is further characterized in that it does not comprise epoxy groups. In a preferred embodiment, the cross-linked material is further characterized in that it does not comprise xenobiotic linker moieties groups.

In a preferred embodiment, the cross-linked material is further characterized in that it does not comprise:
(a) imide groups;
(b) imine groups;
(c) epoxy groups; and/or
(d) xenobiotic linker moieties,
interconnecting one or more fibroin moieties with one or more polysaccharide moieties.

In a preferred embodiment, the cross-linked material is further characterized in that it does not comprise:
(a) imide groups;
(b) imine groups;
(c) epoxy groups; and
(d) xenobiotic linker moieties.

A further aspect of the present invention relates to an injectable composition comprising a cross-linked material of the present invention and a liquid or viscous carrier and optionally further components, preferably wherein the cross-linked material is a hydrogel and/or a super-volumizer.

It will be understood that the definitions and preferred embodiments as laid out in the context of the method and the cross-linked material of the present invention *mutatis mutandis* apply to the injectable composition of the present invention.

In a preferred embodiment, the cross-linked material and/or the injectable composition of the present invention is usable as a soft-tissue filler, in particular a dermal filler or a connective tissue filler.

A liquid or viscous carrier according the present invention as comprised in the injectable composition may be any injectable carrier. Typically, the liquid or viscous carrier is a pharmaceutically and/or cosmetically acceptable carrier, therefore, a carrier that is non-toxic to the mammal, in particular a human, when administered to the mammal in the sense of the present invention. The liquid or viscous carrier may preferably comprise or consist of one or more solvents such as, e.g., water, an aqueous buffer (e.g., a saline or phosphate buffered saline), dimethyl sulfoxide (DMSO), ethanol, vegetable oil, paraffin oil or combinations thereof. More preferably, the liquid or viscous carrier comprises or consists of an apyrogenic isotonic buffer, more particularly a physiological saline solution or a buffered physiological saline solution.

An optionally present further components may be any components. For example, such further component may be selected from the group consisting of one or more local anesthetics, one or more cell proliferation factors, one or more dyes, and combinations of two or more thereof.

Such further components may be added at any time such as before, during or after purifying the cross-linked material. For instance, one or more further components may be added during conducting a purifying step (iii). In another embodiment of the present invention, one or more further components may be added to the prepared and optionally purified cross-linked material.

A local anesthetic may make injection into an individual more comfortable. A cell proliferation factor may improve cellular invasion into an administered cross-linked material of the present invention. A dye may either improve localization of the injection (e.g., a pharmaceutically acceptable fluorescent dye like fluorescein or rhodamine) or may improve invisibility of the otherwise whitish cross-linked material (e.g., by rendering it flesh-colored). Any other pharmaceutically active compound may also be added. Then, the cross-linked material of the present invention may also serve as a retard form for administration.

Suitable local anesthetics for use herein include, but are not limited to, ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and salts thereof. Combinations of two or more of the mentioned anesthetic agents, for example a combination of lidocaine and other "caine"-anesthetic(s) like prilocaine, may also be used herein.

Depending on the intended use of the cross-linked material and the injectable composition of the present invention, it can be provided in different packaging. It may be stored at any condition suitable for this purpose such as, e.g., at ambient temperature (e.g., 18 to 30°C, preferably 18 to 25°C), in a fridge (e.g., at 0 to 15°C, preferably 3 to 10°C), in a freezer (e.g., -30 to 0°C, preferably -25 to -10°C), in a deep freezer (e.g., -100 to -300°C, preferably -90 to -55°C), on liquid nitrogen, on dry ice, or even one or more liquid noble gases. For instance, it may be provided in a vial, in a syringe. It may be administered to a subject via injection (e.g., via a syringe or a drip). It may be stored at dry state, as a hydrogen, as a gel containing other non-aqueous solvents, and/or as a suspension, emulsion, colloid or solution.

Administration may conducted by any means. In a preferred embodiment, administration is administration via a needle. In a preferred embodiment, administration is administration via a syringe, in particular intradermal or subdermal administration via a syringe. Administration may be manual administration, administration using a mechanical pump, or even automated administration. For instance, a Syringe One system may be used for administration.

The cross-linked material of the present invention as well as the injectable composition of the present invention may be used for any purpose. Optionally, the cross-linked material of the present invention as well as the injectable composition of the present invention may be used for cosmetic and/or therapeutic uses. The injectable composition may be a filler, in particular a soft tissue filler such as, e.g., soft-tissue filler, in particular a dermal filler or a connective tissue filler.

As used herein, the term "filler" may be understood in the broadest sense as any agent that can be used to fill a cavity or to serve as a soft tissue filler, preferably a soft tissue filler. A soft tissue filler may be understood in the broadest sense as a material designed to add volume to areas of soft tissue deficiency. A filler may be administered to any location and by any type of injection and may be suitable for uses in cosmetic/anesthetic applications as well as for therapeutic purposes. A filler may generally be any composition that adds, replaces or augments volume under the skin leading to, e.g., smoothened skin wrinkles, augmented lips, improved skin appearance, or treated scars. It is generally used in the dermis area, such as below the epidermis or above the hypodermis and as such may be injected subcutaneously, hypodermically or intradermally, or some combinations.

An injectable composition within the meaning of the present invention may be administered by means of (dispensed from) syringes under normal conditions under normal pressure. Moreover, the filler composition of the present invention is preferably (essentially) sterile. Preferably, the injectable composition is suitable for injection into a mammal, in particular a human.

The present invention also refers to the use of an injectable composition according to the present invention as a filler such as a soft-tissue filler, in particular a dermal filler or a connective tissue filler. It may be used as a super-volumizer. In this context, it may be used as a hydrogel.

The present invention also refers to the use of an injectable composition according to the present invention for cosmetic applications. More preferably, the present invention also refers to the use of an injectable composition according to the present invention for cosmetic applications comprising facial and body re-shaping and rejuvenation.

Accordingly, a further aspect of the present invention relates to the use of an injectable composition of the present invention for cosmetic applications comprising facial and body re-shaping and rejuvenation, preferably including filling of wrinkles, improving facial lines, breast reconstruction or augmentation, rejuvenation of the skin, buttocks augmentation, remodeling of cheekbones, soft-tissue augmentation, filling facial wrinkles, improving glabellar lines, improving nasolabial folds, improving marionette lines, improving buccal commissures, improving peri-lip wrinkles, improving crow's feet, improving subdermal support of the brows, malar and buccal fat pads, improving tear troughs, improving nose appearance, augmentation of lips, augmentation of cheeks, augmentation of peroral region, augmentation of infraorbital region, resolving facial asymmetries, improving jawlines, augmentation of chin, or a combination of two or more thereof.

It will be understood that the definitions and preferred embodiments as laid out in the context of the method, the cross-linked material and the injectable composition of the present invention *mutatis mutandis* apply to the use of the present invention.

In preferred embodiment, the present invention relates to the use of an injectable composition of the present invention for reducing facial folds.

In an embodiment, the use of the present invention may be a cosmetic use, preferably a non-therapeutic use. The use of the present invention may be conducted by cosmetics, cosmetic professionals or health care professionals.

In a preferred embodiment, the use of an injectable composition of the present invention is for improvement of skin quality, treatment of fine lines, treatment of deep lines or volume restauration, or as super-volumizing filler for breast or buttock augmentation.

This use may be a therapeutic and/or cosmetic use. Thus, in other words, the present invention relates to an injectable composition according to the present invention for use in a method for facial and body re-shaping and rejuvenation, preferably including filling of wrinkles, improving facial lines, breast reconstruction or augmentation, rejuvenation of the skin, buttocks augmentation, remodeling of cheekbones, soft-tissue augmentation, filling facial wrinkles, improving glabellar lines, improving nasolabial folds, improving marionette lines, improving buccal commissures, improving peri-lip wrinkles, improving crow's feet, improving subdermal support of the brows, malar and buccal fat pads, improving tear troughs, improving nose appearance, augmentation of lips, augmentation of cheeks, augmentation of peroral region, augmentation of infraorbital region, resolving facial asymmetries, improving jawlines, augmentation of chin, or a combination of two or more thereof.

The present invention also relates to a method of facial and body re-shaping and rejuvenation (preferably including the above specific uses), said method comprising administering the injectable composition according to the present invention.

In other words, the present invention also relates to a method for facial and body re-shaping and rejuvenation, preferably including filling of wrinkles, improving facial lines, breast reconstruction or augmentation, rejuvenation of the skin, buttocks augmentation, remodeling of cheekbones, soft-tissue augmentation, filling facial wrinkles, improving glabellar lines, improving nasolabial folds, improving marionette lines, improving buccal commissures, improving peri-lip wrinkles, improving crow's feet, improving subdermal support of the brows, malar and buccal fat pads, improving tear troughs, improving nose appearance, augmentation of lips, augmentation of cheeks, augmentation of peroral region, augmentation of infraorbital region, resolving facial asymmetries, improving jawlines, augmentation of chin, or a combination of two or more thereof, wherein the a sufficient amount of the injectable composition according to the present invention is administered to a subject in need thereof.

As used herein, a subject (also: an individual) may be any animal, typically a mammal, preferably a domestic mammal or a human. Particularly preferably, an individual is a human. A treated human can also be designated as a patient, independent on his/her health state.

Re-shaping may be performed for cosmetic purposes or may be performed after loss of tissue such as, e.g., caused by an accident or by a surgical intervention. For instance, a part of the face may be injured by an accident. On the other hand, cheekbones may be accentuated by filling the cheekbone region subcutaneously. A breast or part thereof may be surgically removed. On the other hand, breast reconstruction or augmentation may also have aesthetic reasons.

Preferably, the injectable composition of the present invention may be administered in an effective amount to an individual by injection, such as by subcutaneous or intradermal injection. In a preferred embodiment, in the context of this use, the injectable composition is a filler. In a more preferred embodiment, in the context of this use, the injectable composition is a filler, in particular a super-volumizer, and the use comprises the administration of the composition comprising the cross-linked material of the present invention in the tissue of interest, in particular subcutaneously or intradermally. For example, the injectable composition may be intradermally or subcutaneously injected using the serial puncture technique. An effective amount refers to the amount of the (injectable) soft tissue filler composition sufficient to effect beneficial or desired cosmetic (aesthetic) or therapeutic results.

In a particularly preferred embodiment, in the context of this use, the injectable composition is a filler which may be a super-volumizer, in particular a soft tissue filler, and the use comprises administration of the composition comprising the cross-linked material of the present invention subcutaneously or intradermally. For these uses, the cross-linked material according to the present invention is particularly beneficial because the cross-linked material rather stable in aqueous environments such as body fluids and enables invasion of cells due to its structure and characteristics.

A further aspect of the present invention relates to the cross-linked material or the injectable composition according to the present invention for use in a method for regenerating tissue of an individual in need thereof.

In other words, the present invention also relates to a method for regenerating tissue of an individual in need thereof, said method comprising administration of the cross-linked material or the injectable composition according to the present invention to the individual in need thereof. Regenerating tissue of an individual in need thereof may be performed for therapeutic and/or cosmetic purposes.

It will be understood that the definitions and preferred embodiments as laid out in the context of the cross-linked material, the methods and the injectable composition above *mutatis mutandis* apply to the use of regenerating tissue of an individual.

The tissue to be regenerated may be any tissue. In one preferred embodiment, the tissue is a soft tissue. In a more preferred embodiment, the tissue is a soft tissue selected from the group consisting of dermal tissue (including tissue of the dermis and the subcutis) and connective tissue. Then, the method may be used for re-shaping and rejuvenation, including the uses as described above. In another preferred embodiment of the present invention, the tissue is an articulation (joint) tissue. Optionally, for this use, the cross-linked material may comprise one or more cell proliferation factors stimulating proliferation of the respective tissue.

In an alternative preferred embodiment, the tissue is bone tissue. Then, the cross-linked material of the present invention may be administered in a location where bone tissue is intended to grow such as e.g., in a gap of a bone fracture or for elongation of bones. Optionally, for this use, the cross-linked material may comprise one or more cell proliferation factors stimulating bone cell proliferation.

Depending on the specific use, the person skilled in the art will either use particulate cross-linked material according to the present invention or will use a block of the cross-linked material according to the present invention.

For the above therapeutic and cosmetic uses, the cross-linked material according to the present invention is particularly beneficial because the cross-linked material is rather stable in aqueous environments such as body fluids and enables invasion of cells due to its cross-linked structure and surface characteristics.

As indicated above, the cross-linked material of the present invention is obtained by the inventive material obtained when conjugating one or more fibroin moieties with one or more polysaccharide moieties. This conjugate as such also bears unexpectedly beneficial properties.

As used herein, the terms "approximately" and "about" may be understood as a scope including a deviation of up +/- 10% of the respective number value. It will be understood that the specific values are also explicitly disclosed.

It will be further understood that the scope embraces the number values provided as commonly rounded values that embrace the whole rounding limits. For example, the scope of "1 mg" embraces the range of from 0.50 to 1.49 mg.

The number values of the present invention, however, also disclose the more detailed values of one or more orders of magnitude more in detail. Accordingly, for example, "1 mg" may also include the specific disclosure of "1.0 mg".

The examples and claims illustrate embodiments of the present invention.

### Examples

### Materials and Methods

### Raw materials

Hyaluronic acid, different intrinsic viscosities (HTL Biotechnology, Javene, France); 5% aqueous solution of fibroin from silk worm (CareSilk s.r.l.s., Lecce, Italia); 4-(4,6-Dimethoxy-1,3,5-triazin-2-yl)-4-methylmorpholinium chloride (DMTMM) (Sigma Aldrich, Darmstadt, Germany);
Water (internal system for desalted water);

### Lidocaine hydrochloride (Albemarle Corp., Charlotte, USA)

As far as not specified otherwise, all syntheses and measurements are carried out at ambient conditions, i.e., ambient temperature (such as 18 to 25°C, in particular approximately 20°C) and ambient/atmospheric pressure.

### Extrusion Force (EF)

Extrusion Force (EF) was measured using the instrument- TA.XT Plus Texture Analyzer (Stable Micro Systems Ltd., Surrey, UK). A syringe equipped with 30G TSK needle (TSK Laboratory Europe, Oisterwijk, The Netherlands) was placed in the instrument and then the instrument pressed the syringe plunger at constant speed of 0.21 mm/s (approximately 1.26 cm/min) over a distance of 30 mm. The force required to press out the content of the syringe through the needle was recorded, mean value was calculated and this was reported as the Extrusion Force.

### Rheology

Rheology was measured using the instrument- Anton Paar MCR 302 with cone-plate (CP50-1, of 50 mm diameter) geometry (Anton Paar GmbH, Graz, Austria). The measurement was performed in oscillation mode with frequency sweep from 0.1 Hz to 10 Hz at constant deformation of 0.1% at 25°C. Storage modulus (G') and loss factor (tanδ) at 1 Hz were reported as the measurement results.

### Example 1 - Preparation of cross-linked material and influence of the amount of lubrication phase on the properties of the material

Hyaluronic acid (HA) with intrinsic viscosity of 2.8 m³/kg (4.6 g corresponding to 4.0 g of dry polymer) was dissolved in 200 g of water (polymer concentration was 20 mg/g). DMTMM (3.3 g corresponding to 2.8 g of dry material - 1 eq. regarding the amount of HA) was added and the mixture was stirred for 1 hour. Thereafter, 100 mL of fibroin solution (concentration was 20 mg/g, HA/fibroin weight ratio was 2/1) was added: The mixture was stirred for 2 hours and then stirring was stopped. On the next day, the cross-linked material (here exemplified as a HA/fibroin gel) was purified using dynamic crossflow filtration (DCF Andritz, Membrane d= 152 mm (Andritz AG, Graz, Austria)) and 5 mM phosphate-buffered saline (PBS) solution at room temperature. The purified cross-linked material (here exemplified as a HA/fibroin gel) was then mixed with different amounts of lubrication phase (lubrication phase had concentration of 30 mg/g). Finally, the cross-linked material (here exemplified as a HA/fibroin gel) was filled into 1 mL syringes (Syringe One) and sterilized at 127°C for 8 min. Results are depicted in Table 1 below.

**Table 1. Properties of the obtained cross-linked material (here exemplified as a HA/fibroin gel). All given values are mean of double determination**

| Batch name | Amount of lubrication phase [%] | G' at 1 Hz [Pa] | | G' drop [%] | tanδ at 1 Hz | | Extrusion Force (30G TSK needle) [N] |
|---|---|---|---|---|---|---|---|
| (before or after) sterilization | | before | after | after | before | after | |
| Fib01A | 0 | 300 | 278 | 8 | 0.146 | 0.142 | 11.7 |
| Fib01B | 10 | 256 | 216 | 16 | 0.268 | 0.280 | 10.5 |
| Fib01C | 20 | 288 | 225 | 22 | 0.321 | 0.393 | 12.5 |
| Fib01D | 30 | 372 | 301 | 19 | 0.399 | 0.467 | 13.0 |
| Fib01E | 50 | 348 | 260 | 25 | 0.469 | 0.563 | 13.2 |
| Fib01F | 100 (control) | 1407 | 309 | 78 | 0.326 | 1.054 | 13.2 |

G' drop was calculated using the following formula:
G' drop = (G' at 1 Hz before sterilization - G' at 1 Hz after sterilization) / G' at 1 Hz before sterilization

Lubrication phase may be added to gels to reduce the extrusion force, however, in case of the cross-linked materials (here exemplified as a HA/fibroin gels) according to the present invention, the lubrication phase does surprisingly not cause a decrease of the extrusion force. Therefore, other materials might be prepared without lubrication phase.

It was found that a cross-linked material (here exemplified as a HA/fibroin gel) without lubrication phase may have G' comparable to or even higher than the commercially available cross-linked hyaluronan product Belotero Volume Lidocaine (Anteis S.A., Plan-les-Ouates, Switzerland). While one of the herein exemplified HA/fibroin gels had a G' of approximately 278 Pa after sterilization (Fib01A), comparable a hyaluronan product Belotero Volume Lidocaine had a G' of approximately 270 Pa. But extrusion force (EF) through 30G TSK needle was found to be significantly lower. The investigated HA/fibroin gel had an extrusion force (EF) of approximately 12 N, while cross-linked hyaluronan (Belotero Volume Lidocaine) had an extrusion force (EF) of approximately 22 N. This may support that HA/fibroin gels could offer similar lifting effect like the commercially available cross-linked hyaluronan product Belotero Volume Lidocaine (because of similar G'), while offering even better injection performance for the practitioner.

Furthermore, in this set of experiments the G' of the cross-linked materials (here exemplified as a HA/fibroin gels) decreases by 10-25% after sterilization, which is significantly lower than the decrease of G' of 78% in case of the control experiment (pure non-cross-linked hyaluronan). The results are also shown in Table 1 above.

### Example 2 - Preparation of cross-linked material and influence of HA/fibroin ratio on material properties)

Hyaluronic acid (HA) with intrinsic viscosity of 2.8 m³/kg (3.5 g corresponding to 3.0 g of dry polymer) was dissolved in 150 g of water (polymer concentration was 20 mg/g). DMTMM (2.5 g corresponding to 2.1 g of dry material - 1 eq. in regard to the amount of HA) was added and the mixture was stirred for 1 hour. Thereafter, 150 mL of fibroin solution (concentration was 20 mg/g, HA/fibroin weight ratio was 1/1) was added. The mixture was stirred for 2 hours and then stirring was stopped. On the next day, the cross-linked material (here exemplified as a HA/fibroin gel) was purified using dynamic crossflow filtration (DCF Andritz, Membrane d= 152 mm (Andritz AG, Graz, Austria)) and 5 mM phosphate-buffered saline (PBS) solution at room temperature. No lubrication phase was added. Finally, the cross-linked material (here exemplified as a HA/fibroin gel) was filled into 1 mL syringes (Syringe One) and sterilized at 127°C for 8 min. Results are depicted in Table 2 below.

**Table 2. Properties of the obtained cross-linked material (here exemplified as a HA/fibroin gel). All given values are mean of double determination**

| Batch name | HA/Fibroin weight ratio | G' at 1 Hz [Pa] | tanδ at 1 Hz | Extrusion Force (30G TSK needle) [N] | | |
|---|---|---|---|---|---|---|
| (before or after) sterilization | | before | after | before | after | |
| Fib01A | 2/1 | 300 | 278 | 0.146 | 0.142 | 11.7 |
| Fib05 | 1/1 | 634 | 531 | 0.272 | 0.221 | 7.6 |

It was found that an increase of the amount of fibroin in the cross-linked material, leads to cross-linked materials (here exemplified as a HA/fibroin gels) with higher G'.

Notably, this cross-linked material has lower extrusion force (even though it has higher G'). Explanation for this could be thixotropic (shear-thinning) behavior of fibroin. Cross-linked materials with comparably high and adjustable G' and comparably low and adjustable extrusion force can be obtained.

### Example 3 - Preparation of cross-linked material and influence of intrinsic viscosity (IV) of hyaluronic acid (HA) on the material properties

Hyaluronic acid (HA) with intrinsic viscosity of 1.5 m³/kg (3.5 g corresponding to 3.0 g of dry polymer) was dissolved in 150 g of water (polymer concentration was 20 mg/g). DMTMM (2.5 g corresponding to 2.1 g of dry material - 1 eq. in regard to the amount of HA) was added and the mixture was stirred for 1 hour. Thereafter, 150 mL of fibroin solution (concentration was 20 mg/g, HA/fibroin weight ratio was 1/1) was added. The mixture was stirred for 2 hours and then stirring was stopped. On the next day, the cross-linked material (here exemplified as a HA/fibroin gel) was purified using dynamic crossflow filtration (DCF ANDRITZ, Membrane d= 152 mm (Andritz AG, Graz, Austria)) and 5 mM phosphate-buffered saline (PBS) solution at room temperature. No lubrication phase was added. Finally, the cross-linked material (here exemplified as a HA/fibroin gel) was filled into 1 mL syringes (Syringe One) and sterilized at 127°C for 8 min. Results are depicted in Table 3 below.

**Table 3. Properties of the obtained cross-linked material (here exemplified as a HA/fibroin gel). All given values are mean of double determination**

| Batch name | Intrinsic viscosity (IV) of HA [m³/kg] | G' at 1 Hz [Pa] | tanδ at 1 Hz | Extrusion Force (30G TSK needle) [N] | | |
|---|---|---|---|---|---|---|
| (before or after) sterilization | | before | after | before | after | |
| Fib05 | 2.8 | 634 | 531 | 0.272 | 0.221 | 7.6 |
| Fib06 | 1.5 | 373 | 312 | 0.162 | 0.137 | 6.0 |

### Example 4 - Enzymatic degradation of HA/fibroin gels

In order to investigate whether cross-linked materials (herein exemplified as HA/fibroin gels) can also be used as a reversible filler, the materials were treated with the enzyme hyaluronidase from ovine testes. Namely, approximately 0.50 g of gel was weighed via differential weighting and placed on the plate of the CP50-1 (cone-plate) system of Anton Paar MCR 302 rheometer (Anton Paar GmbH, Graz, Austria). A homogenous aqueous solution of 150 µL WFI containing 50 U hyaluronidase was added on top of the hydrogel on the plate. The hyaluronidase hydrogel mixture was homogenized manually by, e.g., the pipette tip for approximately 10 seconds. Thereafter, measurement was performed at 37°C in the oscillation mode at a deformation of 0.1% and a frequency of 1 Hz. Measurement duration was 60 minutes with recording 1 point/min.

A gel prepared of non-cross-linked HA was degraded fastest. Thereafter, was the gel consisting of cross-linked HA (Belotero Volume, Anteis S.A., Plan-les-Ouates, Switzerland ) and the gels containing fibroin degraded slowest. This could be indication of prolonged longevity of gel which contain fibroin compared to typical HA cross-linked gels. Results are depicted in Table 4 below.

**Table 4. Enzymatic degradation of hydrogels (here exemplified as a HA/fibroin gel). All given values are mean of double determination**

| Batch name | HA/Fibroin ratio | G' at 1 Hz before treatment [Pa] | G' at 1 Hz after treatment with hyaluronidase (at 10 min) [Pa] | G' drop [%] |
|---|---|---|---|---|
| Fib01A | 2/1 | 531 | 207 | 61 |
| Fib05 | 1/1 | 278 | 107 | 62 |
| Belotero Volume Lidocaine | Pure HA-cross-linked | 235 | 48 | 80 |
| Fib01F | Pure HA-non-cross-linked | 309 | 9 | 97 |

G' drop was calculated using the following formula:
G' drop = (G' at 1 Hz before treatment - G' at 1 Hz after treatment with hyaluronidase (at 10 min)) / G' at 1 Hz before treatment

### Example 5 -Accelerated stability study

To test stability, batch Fib05 (see above) was mixed with lidocaine (to prepare new batch: Fib05L) and placed in a climate chamber at 40°C (accelerated conditions). Characterization was carried out by measuring rheological properties and extrusion force (using 30G TSK needle) of the gel at different time points (at week 4, 8 and 12). All measurements were performed in triplicates. The results are depicted in Table 5 below.

**Table 5. Properties of the obtained cross-linked material (here exemplified as a HA/fibroin gel) over time, incubated at 40°C at 75% relative humidity**

| Parameter | T0 | 4 weeks | 8 weeks | 12 weeks |
|---|---|---|---|---|
| G' [Pa] | 440 | 442 | 483 | 434 |
| tanδ | 0.251 | 0.193 | 0.212 | 0.217 |
| EF (30G TSK needle ) [N] | 9.5 | 9.3 | 9.2 | 8.0 |

It was found that the cross-linked materials (herein exemplified as HA/fibroin gels) are comparably stable over time. Even after 12 weeks at 40°C at 75% relative humidity, no significant degradation or deterioration of properties were found.

In summary, it was found that cross-linked materials of the present invention can be very well and efficiently prepared, optionally in a single batch, without burden. The materials have good properties to be injectable and appear to have shear-thinning / thixotropic properties. The materials have a comparably high viscosity and are comparably high biological /enzymatic stability. These properties enable the cross-linked materials of the present invention to be particularly usable as soft-tissue filers such as dermal or connective tissue fillers. The materials are well storable and has a comparatively high shelf-life.

The invention can further be characterized by the following embodiments:
Embodiment 1. A method for preparing a cross-linked material, said method comprising:
   (i) contacting the following components with each other:
      (A) one or more fibroin moieties comprising primary amino residues or salts thereof,
      (B) one or more polysaccharide moieties comprising carboxylic acid residues or salts thereof,
      (C) one or more activating agents that effect reaction of carboxylic acid residues with amino residues thereby forming amide bonds, and
      (D) one or more solvents; and
   (ii) allowing a reaction of at least some of the carboxylic acid residues with at least some of the primary amino residues to form amide bonds conjugating the one or more fibroin moieties covalently with the one or more polysaccharide moieties; and
   (iii) optionally purifying the cross-linked material obtained from step (ii).
Embodiment 2. The method of embodiment 1, wherein the one or more fibroin moieties have a weight average molecular weight of at least 5 kDa, in the range of 5 to 1000 kDa, in the range of 5 to 400 kDa, in the range of 10 to 400 kDa, or in the range of 100 to 150 kDa, preferably wherein the one or more fibroin moieties are silk fibroin moieties, more preferably silk fibroin moieties having at least having at least 80% sequence homology to a natural insect or spider silk fibroin moiety.
Embodiment 3. The method of any of embodiments 1 or 2, wherein the one or more polysaccharide moieties comprise or consist of hyaluronic acid, heparosan, chondroitin sulfate, and carboxymethyl cellulose.
Embodiment 4.The method of any of embodiments 1 to 3, wherein the one or more polysaccharide moieties comprise or consist of one or more hyaluronic acid moieties.
Embodiment 5. The method of any of embodiments 1 to 4, wherein the one or more polysaccharide moieties have a weight average molecular weight of at least 50 kDa, in particular in the range of 50 to 4000 kDa.
Embodiment 6. The method of any of embodiments 1 to 5, wherein the mass ratio between the one or more fibroin moieties (A) and the one or more polysaccharide moieties (B) A : B is in the range of 5:1 to 1:20, preferably in the range of 1:1 to 1:10, in particular in the range of 1:1 to 1:5.
Embodiment 7.The method of any of embodiments 1 to 6, wherein the one or more activating agents are selected from the group consisting of
   (C1) one or more triazine-based activating agents, in particular selected from the group consisting of 4-(4,6-dimethoxy-1,3,5-triazin-2-yl)-4-methylmorpholinium, a salt thereof, and/or 2-chloro-4,6,-dimethoxy-1,3,5-triazine, and combinations thereof;
   (C2) one or more carbodiimide activating agents, in particular selected from the group consisting of N,N'-dicyclohexylcarbodiimide, diisopropylcarbodiimide, 1-ethyl-3-(3-dimethylaminopropyl)-carbodiimid, and combinations of two or more thereof; and
   (C3) combinations thereof.
Embodiment 8.The method of any of embodiments 1 to 7, wherein the activating agent is 4-(4,6-dimethoxy-1,3,5-triazin-2-yl)-4-methylmorpholinium or a salt thereof, in particular 4-(4,6-dimethoxy-1,3,5-triazin-2-yl)-4-methylmorpholinium chloride.
Embodiment 9. The method of any of embodiments 1 to 8, wherein the method is further characterized in that:
   (a) it comprises a step (iii) of purifying the cross-linked material by filtration, washing and/or dialysis, in particular crossflow filtration, diafiltration and/or dead-end filtration;
   (b) steps (i) and (ii) are conducted in a single batch; and/or
   (c) steps (i) and (ii) and optional step (iii) are conducted at a temperature in the range of from 5 to 90°C, in particular 18 to 30°C.
Embodiment 10. The method of any of embodiments 1 to 9, wherein the method comprises:
   (i) contacting the following components with each other:
      (A) one or more silk fibroin moieties having a weight average molecular weight of at least 5 kDa that comprises primary amino residues or salts thereof,
      (B) one or more hyaluronic acid moieties having a weight average molecular weight of at least 50 kDa that comprises carboxylic acid residues or salts thereof,
      (C) one or more triazine-based activating agents that promote effect thereby forming amide bonds, in particular wherein the activating agent is 4-(4,6-dimethoxy-1,3,5-triazin-2-yl)-4-methylmorpholinium or a salt thereof; and
      (D) one or more solvents; and
   (ii) allowing a reaction of at least some of the carboxylic acid residues with at least some of the primary amino residues to form amide bonds conjugating the one or more silk fibroin moieties covalently with the one or more hyaluronic acid moieties; and
   (iii) optionally purifying the cross-linked material obtained from step (ii).
Embodiment 11. A cross-linked material obtainable from a method of any of embodiments 1 to 10.
Embodiment 12. A cross-linked material comprising or consisting of
   (A) one or more fibroin moieties having a weight average molecular weight of at least 5 kDa, and
   (B) one or more hyaluronic acid moieties having a weight average molecular weight of at least 50 kDa,
   wherein the one or more fibroin moieties are covalently conjugated with the with the one or more hyaluronic acid moieties via amide bonds without an interconnecting linker structure.
Embodiment 13The cross-linked material of any of embodiments 11 or 12, wherein said cross-linked material is further characterized in that it does not comprise:
   (a) imide groups;
   (b) imine groups;
   (c) epoxy groups; and
   (d) xenobiotic linker moieties.
Embodiment 14.An injectable composition comprising a cross-linked material of any of embodiments 10 to 12 and a liquid or viscous carrier and optionally further components, preferably wherein the cross-linked material is a hydrogel and/or a super-volumizer.
Embodiment 15.Use of an injectable composition of embodiment 14 for cosmetic applications comprising facial and body re-shaping and rejuvenation, preferably including filling of wrinkles, improving facial lines, breast reconstruction or augmentation, rejuvenation of the skin, buttocks augmentation, remodeling of cheekbones, soft-tissue augmentation, filling facial wrinkles, improving glabellar lines, improving nasolabial folds, improving marionette lines, improving buccal commissures, improving peri-lip wrinkles, improving crow's feet, improving subdermal support of the brows, malar and buccal fat pads, improving tear troughs, improving nose appearance, augmentation of lips, augmentation of cheeks, augmentation of peroral region, augmentation of infraorbital region, resolving facial asymmetries, improving jawlines, augmentation of chin, or a combination of two or more thereof.

## Claims

1. A method for preparing a cross-linked material, said method comprising:
(i) contacting the following components with each other:
(A) one or more fibroin moieties comprising primary amino residues or salts thereof,
(B) one or more polysaccharide moieties comprising carboxylic acid residues or salts thereof,
(C) one or more activating agents that effect reaction of carboxylic acid residues with amino residues thereby forming amide bonds, and
(D) one or more solvents; and
(ii) allowing a reaction of at least some of the carboxylic acid residues with at least some of the primary amino residues to form amide bonds conjugating the one or more fibroin moieties covalently with the one or more polysaccharide moieties; and
(iii) optionally purifying the cross-linked material obtained from step (ii).

2. The method of claim 1, wherein at least a part of the one or more fibroin moieties comprises one or more lysyl residues and the amino groups form part of the lysyl residues.

3. The method of claim 1 or 2, wherein the polysaccharide has not been oxidized.

4. The method of any of claims 1 to 3, wherein the one or more fibroin moieties have a weight average molecular weight of at least 5 kDa, in the range of 5 to 1000 kDa, in the range of 5 to 400 kDa, in the range of 10 to 400 kDa, or in the range of 100 to 150 kDa, preferably wherein the one or more fibroin moieties are silk fibroin moieties, more preferably silk fibroin moieties having at least having at least 80% sequence homology to a natural insect or spider silk fibroin moiety.

5. The method of any of claims 1 to 4, wherein the one or more polysaccharide moieties are selected from the group consisting of hyaluronic acid, heparosan, chondroitin sulfate, and carboxymethyl cellulose, preferably comprise or consist of one or more hyaluronic acid moieties.

6. The method of any of claims 1 to 5, wherein the one or more polysaccharide moieties have a weight average molecular weight of at least 50 kDa, in particular in the range of 50 to 4000 kDa.

7. The method of any of claims 1 to 6, wherein the mass ratio between the one or more fibroin moieties (A) and the one or more polysaccharide moieties (B) A : B is in the range of 5:1 to 1:20, preferably in the range of 1:1 to 1:10, in particular in the range of 1:1 to 1:5.

8. The method of any of claims 1 to 7, wherein the one or more activating agents are selected from the group consisting of
(C1) one or more triazine-based activating agents, in particular selected from the group consisting of 4-(4,6-dimethoxy-1,3,5-triazin-2-yl)-4-methylmorpholinium, a salt thereof, and/or 2-chloro-4,6,-dimethoxy-1,3,5-triazine, and combinations thereof, preferably 4-(4,6-dimethoxy-1,3,5-triazin-2-yl)-4-methylmorpholinium or a salt thereof, in particular 4-(4,6-dimethoxy-1,3,5-triazin-2-yl)-4-methylmorpholinium chloride;
(C2) one or more carbodiimide activating agents, in particular selected from the group consisting of N,N'-dicyclohexylcarbodiimide, diisopropylcarbodiimide, 1-ethyl-3-(3-dimethylaminopropyl)-carbodiimid, and combinations of two or more thereof; and
(C3) combinations thereof.

9. The method of any of claims 1 to 8, wherein the method is further **characterized in that**:
(a) it comprises a step (iii) of purifying the cross-linked material by filtration, washing and/or dialysis, in particular crossflow filtration, diafiltration and/or dead-end filtration;
(b) steps (i) and (ii) are conducted in a single batch; and/or
(c) steps (i) and (ii) and optional step (iii) are conducted at a temperature in the range of from 5 to 90°C, in particular 18 to 30°C.

10. The method of any of claims 1 to 9, wherein the method comprises:
(i) contacting the following components with each other:
(A) one or more silk fibroin moieties having a weight average molecular weight of at least 5 kDa that comprises primary amino residues or salts thereof,
(B) one or more hyaluronic acid moieties having a weight average molecular weight of at least 50 kDa that comprises carboxylic acid residues or salts thereof,
(C) one or more triazine-based activating agents that promote effect thereby forming amide bonds, in particular wherein the activating agent is 4-(4,6-dimethoxy-1,3,5-triazin-2-yl)-4-methylmorpholinium or a salt thereof; and
(D) one or more solvents; and
(ii) allowing a reaction of at least some of the carboxylic acid residues with at least some of the primary amino residues to form amide bonds conjugating the one or more silk fibroin moieties covalently with the one or more hyaluronic acid moieties; and
(iii) optionally purifying the cross-linked material obtained from step (ii).

11. A cross-linked material obtainable from a method of any of claims 1 to 10.

12. A cross-linked material comprising or consisting of
(A) one or more fibroin moieties having a weight average molecular weight of at least 5 kDa, and
(B) one or more hyaluronic acid moieties having a weight average molecular weight of at least 50 kDa,
wherein the one or more fibroin moieties are covalently conjugated with the with the one or more hyaluronic acid moieties via amide bonds without an interconnecting linker structure, wherein the cross-linked material does not comprise imine groups.

13. The cross-linked material of any of claims 11 or 12, wherein said cross-linked material is further **characterized in that** it does not comprise:
(a) imide groups;
(b) imine groups;
(c) epoxy groups; and
(d) xenobiotic linker moieties.

14. An injectable composition comprising a cross-linked material of any of claims 11 to 12 and a liquid or viscous carrier and optionally further components, preferably wherein the cross-linked material is a hydrogel and/or a super-volumizer.

15. Use of an injectable composition of claim 14 for cosmetic applications comprising facial and body re-shaping and rejuvenation, preferably including filling of wrinkles, improving facial lines, breast reconstruction or augmentation, rejuvenation of the skin, buttocks augmentation, remodeling of cheekbones, soft-tissue augmentation, filling facial wrinkles, improving glabellar lines, improving nasolabial folds, improving marionette lines, improving buccal commissures, improving peri-lip wrinkles, improving crow's feet, improving subdermal support of the brows, malar and buccal fat pads, improving tear troughs, improving nose appearance, augmentation of lips, augmentation of cheeks, augmentation of peroral region, augmentation of infraorbital region, resolving facial asymmetries, improving jawlines, augmentation of chin, or a combination of two or more thereof.
